# EUROPÄISCHE PATENTSCHRIFT

(11) **EP 2 475 319 B1**
(45) Veröffentlichungstag und Bekanntmachung des Hinweises auf die Patenterteilung: **11.01.2017**
(21) Anmeldenummer: 10745169.2
(22) Anmeldetag: 12.08.2010
(51) Int. Cl.: A61B 18/12, A61B 17/00, A61B 5/00, A61B 5/053, A61B 18/00

(54) **HF-CHIRURGIEGERÄT**
HF SURGICAL DEVICE
APPAREIL CHIRURGICAL HF

(30) Priorität: 11.09.2009 DE 102009041169; 13.10.2009 DE 102009049180
(43) Veröffentlichungstag der Anmeldung: 18.07.2012
(73) Patentinhaber: ERBE Elektromedizin GmbH, 72072 Tübingen (DE)
(72) Erfinder: SELIG, Peter, 72379 Hechingen (DE); FRITZ, Martin, 72070 Tübingen (DE)
(74) Vertreter: Rüger, Barthelt & Abel
(86) Internationale Anmeldenummer: PCT/EP2010/004953
(87) Internationale Veröffentlichungsnummer: WO 2011/029509

(56) Entgegenhaltungen:
- EP-A1- 1 803 410
- EP-A2- 1 051 948
- US-B1- 6 186 147

## Beschreibung

Die Erfindung betrifft ein HF-Chirurgiegerät mit einer automatischen Start-Funktion zum Schneiden und/oder Koagulieren von biologischem Gewebe sowie, ein Verfahren zum automatischen Starten eines HF-Stroms eines HF-Chirurgiegeräts.

Es gibt HF-Chirurgiegeräte mit einer automatischen Startfunktion. Hierbei wird der HF-Strom mit den voreingestellten Parametern aktiviert, sobald ein ausreichender Kontakt zwischen Instrument und dem Gewebe besteht. Dies ist sowohl bei bipolaren als auch bei monopolaren Anwendungen möglich. Das Gerät kann so konfiguriert werden, dass bei Berührung des Gewebes mit einer Pinzette oder Klemme und einem daraus resultierenden Unterschreiten einer voreingestellten Impedanzschwelle (nach Ablauf einer definierten Zeitverzögerung) der HF-Strom automatisch aktiviert wird.

Bisher bekannte Systeme weisen zwei Nachteile bei diesem Verfahren auf: In der Regel beinhalten die Geräte entweder eine separate Sensorik zur Bestimmung der Impedanz zwischen den Anschlüssen, oder die Messtechnik des HF-Generators, die normalerweise zur Ermittlung der aktuellen HF-Betriebsdaten dient, wird hierfür in Kombination mit einem HF-Generator als Messstromquelle genutzt.

Ist letzteres der Fall, so wird der HF-Generator mit minimalen Spannungs- und Stromwerten permanent aktiviert, auf die Ausgänge geschaltet und das HF-Signal als Messgröße für die Impedanz herangezogen. Vorteil ist hier, dass die Messtechnik des HF-Generators typischerweise sehr präzise Messungen ermöglicht, gravierender Nachteil ist allerdings, dass die Messsignale mit Spannungsamplituden von einigen Zehn Volt bauartbedingt relativ groß sind. Damit ergibt sich ein permanentes Störpotential für empfindliche Geräte in der Umgebung, wie z.B. EKG-Monitore.

Außerdem kann die Autostart-Funktionalität immer nur an einem einzigen Ausgang des Geräts eingeschaltet werden, da bei der Parallelschaltung mehrerer Ausgänge keine Unterscheidung möglich ist, welcher Ausgang den entscheidenden Teil zur Gesamtimpedanz beiträgt. Eine schnelle Unischaltung des Signals zwischen verschiedenen Ausgängen kommt aufgrund der zu schaltenden Relais nicht in Frage.

Wird hingegen eine separate Sensorik für die Autostart-Funktionalität verwendet (vgl. z.B. EP 1051948 B1), so können die Messsignale in ihrer Amplitude wesentlich geringer gehalten werden. Allerdings ist neben der Messtechnik des HF-Generators eine weitere sehr genaue Sensorik erforderlich, welche die Impedanz am Ausgang überwacht. Prinzipiell kann dies an mehreren Ausgängen parallel realisiert werden, womit die Funktion an mehreren Ausgängen zeitgleich ausgewählt werden kann. Allerdings ist dies, bedingt durch die erforderliche Genauigkeit der Sensorik, teuer und wirtschaftlich ungünstig.

Der Erfindung liegt die Aufgabe zugrunde, ein HF-Chirurgiegerät der eingangs genannten Art dahingehend aufzuzeigen, das in kostengünstiger Weise einerseits eine automatische Startfunktion an mehreren Anschlüssen gleichzeitig und andererseits eine präzise Feststellung eines Kontakts zwischen Elektrode und Gewebe ermöglicht.

Diese Aufgabe wird durch ein HF-Chirurgiegerät nach Anspruch 1 gelöst.

Ein wesentlicher Punkt der Erfindung besteht darin, dass nach einem Feststellen eines Kontakts zwischen Gewebe und einer Elektrode mit Hilfe von jedem Ausgang zugeordneten ersten Messeinrichtungen das Feststellen des Kontakts zwischen Gewebe und Elektrode mit Hilfe einer zweiten Messeinrichtung überprüft wird und der HF-Strom für den jeweiligen Ausgang erst dann eingeschaltet wird, wenn das Feststellen des Kontakts zwischen Gewebe und Elektrode mit Hilfe der zweiten Messeinrichtung bestätigt wurde, die in dem HF-Generator zum Erzeugen des HF-Stroms angeordnet ist. Dadurch können die ersten Messeinrichtüngen kostengünstiger als die zweite Messeinrichtung sein, da bei einer ungenauen bzw. falschen Messung durch die ersten Messeinrichtungen der Strom aufgrund des zweiten Messvorgangs mit der zweiten Messeinrichtung nicht eingeschaltet wird.

Die zweite Messeinrichtung bestimmt einen genaueren Messwert der Impedanz der jeweiligen an dem ausgewählten Ausgang angeschlossenen Elektrode als die jeweilige erste Messeinrichtung. Ein Vorteil hiervon ist, dass die erste Messeinrichtung sehr viel einfacher als die zweite Messeinrichtung sein kann.

Die ersten Messeinrichtungen verwenden im Vergleich zur zweiten Messeinrichtung eine geringere Spannung zur Messung der Impedanz. Hierdurch werden Störungen anderer Geräte in der Nähe des HF-Chirurgiegeräts durch elektromagnetische Strahlung, Spannungsabfälle/-spitzen im Leitungsnetz oder dem Messstrom selbst gemindert.

In einer weiteren Ausführungsform umfasst das HF-Chirurgiegerät einen dritten Komparator zum Vergleichen des zweiten Messwerts mit einem dritten Sollwert und zum Ausschalten des HF-Stroms und der zweiten Messeinrichtung und zum Einschalten der ersten Messeinrichtung, wenn der zweite Messwert größer als der oder gleich dem dritten Sollwert ist. Hierdurch kann die am ausgewählten Ausgang angeschlossene Elektrode so lange mit HF-Strom versorgt werden, bis der Kontakt mit dem Gewebe gelöst ist und daher der zweite Messwert größer als der oder gleich dem dritten Sollwert ist. Das HF-Chirurgiegerät wird dann wieder in den Grundzustand zurückversetzt.

Das HF-Chirurgiegerät kann einen vierten Komparator zum Vergleichen des zweiten Messwerts mit einem vierten Sollwert und zum Ausschalten der zweiten Messeinrichtung und zum Einschalten der ersten Messeinrichtung, wenn der zweite Messwert größer als der oder gleich dem vierten Sollwert ist, umfassen. Ein Vorteil hiervon ist, dass der zweite Messwerts wiederholt gemessen werden kann, solange der zweite Messwert größer als der oder gleich dem zweiten Sollwert und kleiner als der vierte Sollwert ist, wenn also unklar ist, ob das Gewebe nur kurz oder nur leicht durch die Elektrode berührt wurde.

Der dritte und der vierte Sollwert können gleich groß sein. Hierdurch können die Vergleiche des zweiten Messwerts mit dem dritten bzw. vierten Sollwert vereinfacht werden und die Schwelle, bei dem das HF-Chirurgiegerät in den Grundzustand zurückversetzt wird, ist unabhängig davon, ob der zweite Messwert je unterhalb des zweiten Sollwerts lag.

In einer weiteren Ausführungsform sind die ersten Messeinrichtungen derart ausgebildet, dass sie die ersten Messwerte in zueinander disjunkten Zeitintervallen messen. Hierdurch werden Störungen der Messeinrichtungen untereinander durch Querströme zwischen den Elektroden weitgehend verhindert.

Die zweite Messeinrichtung kann in dem HF-Generator zum Erzeugen des HF-Stroms angeordnet sein. Hierdurch können die Verbindungswege zwischen der zweiten Messeinrichtung und dem HF-Generator weitgehend minimiert werden. Zudem kann die zweite Messeinrichtung zur Überwachung der HF-Betriebsdaten verwendet werden.

Ein erfindungsgemäßes Verfahren zum automatischen Starten eines HF-Stroms eines HF-Chirurgiegeräts umfasst das Einschalten von einer dem Ausgang zugeordneten ersten Messeinrichtung, das Messen eines ersten Messwerts einer Impedanz der am Ausgang angeschlossenen Elektrode mit der ersten Messeinrichtung, das Vergleichen des gemessenen ersten Messwerts mit einem vorher bestimmten ersten Sollwert, das Ausschalten der ersten Messeinrichtung und das Einschalten einer zweiten Messeinrichtung für den Ausgang, wenn der erste Messwert kleiner als der vorher bestimmte erste Sollwert ist, das Messen eines zweiten Messwerts der Impedanz der an dem Ausgang angeschlossenen Elektrode mit der zweiten Messeinrichtung, das Vergleichen des zweiten Messwerts mit einem zweiten Sollwert und das Einschalten des HF-Stroms für den Ausgang, wenn der zweite Messwert kleiner als der vorher bestimmte zweite Sollwert ist.

Ein weiteres erfindungsgemäßes Verfahren automatischen Starten eines HF-Stroms eines HF-Chirurgiegeräts umfasst das Einschalten von jeweils einer dem jeweiligen Ausgang zugeordneten ersten Messeinrichtung, das Messen eines jeweiligen ersten Messwerts einer Impedanz der am jeweiligen Ausgang angeschlossenen Elektrode mit den ersten Messeinrichtungen, das Vergleichen des jeweils gemessenen ersten Messwerts mit einem vorher bestimmten ersten Sollwert, das Auswählen eines Ausganges, bei dem der erste Messwert als zeitlich frühester der ersten Messwerte kleiner als der vorher bestimmte erste Sollwert ist, das Ausschalten der ersten Messeinrichtung und Einschalten einer zweiten Messeinrichtung für den ausgewählten Ausgang, das Messen eines zweiten Messwerts der Impedanz der an dem ausgewählten Ausgang angeschlossenen Elektrode mit der zweiten Messeinrichtung, das Vergleichen des zweiten Messwerts mit einem zweiten Sollwert und das Einschalten des HF-Stroms für den ausgewählten Ausgang, wenn der zweite Messwert kleiner als der vorher bestimmte zweite Sollwert ist.

Bevorzugte Ausführungsformen der Erfindung ergeben sich aus den Unteransprüchen sowie aus der nachstehenden Beschreibung von Ausführungsbeispielen, die anhand von Abbildungen näher erläutert werden. Hierbei zeigen
- - Fig. 1: eine schematische Ansicht einer Schaltung für ein HF-Chirurgiegerät,
- - Fig. 2: ein Flussdiagramm für den Betrieb eines HF-Chirurgiegeräts mit einer Autostart-Funktion,
- - Fig. 3: eine schematische Ansicht einer Schaltung für ein HF-Chirurgiegeräts während der Messung der Impedanz mit den ersten Messeinrichtungen,
- - Fig. 4: die Schaltung nach Fig. 3 während der Messung der Impedanz mit der zweiten Messeinrichtung bzw. während des eingeschalteten HF-Stroms und
- - Fig. 5: ein Flussdiagramm für den Betrieb der Schaltung nach Fig. 3 bzw. Fig. 4.

Bei der nachfolgenden Beschreibung werden für gleiche und gleich wirkende Teile dieselben Bezugsziffern verwendet.

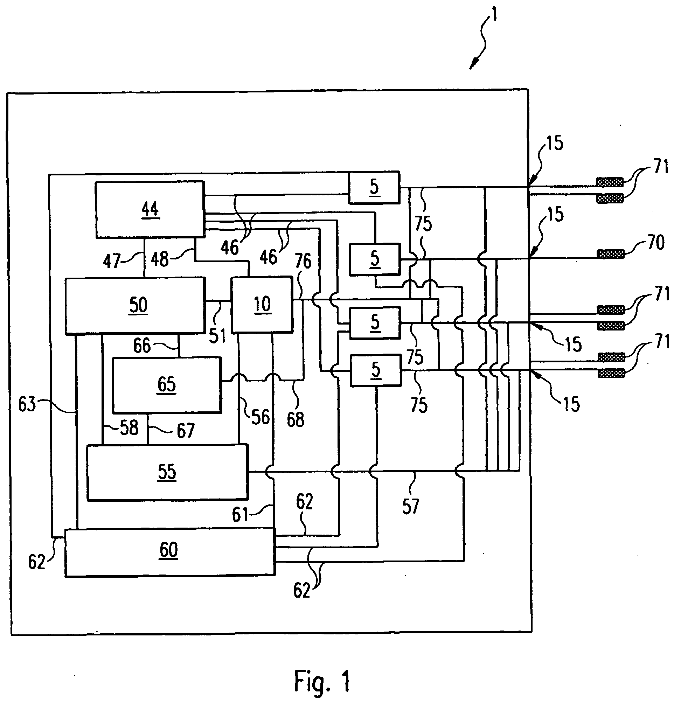

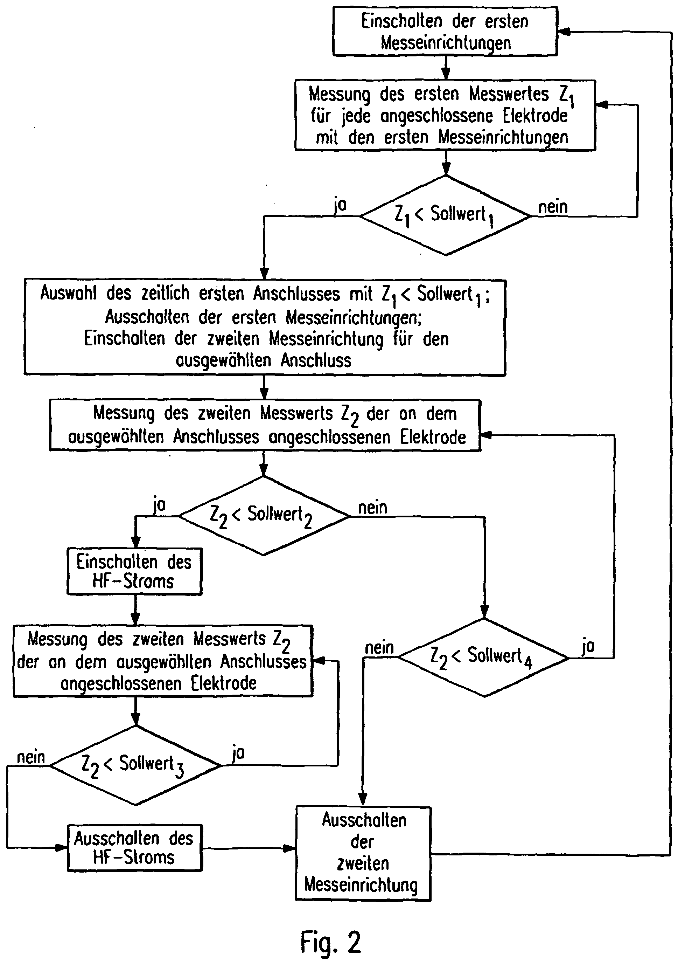

Fig. 1 zeigt eine schematische Ansicht einer Schaltung 1 für ein HF-Chirurgiegerät mit Autostart-Funktion. Fig. 2 zeigt ein Flussdiagramm für ein Verfahren zum Betrieb eines HF-Chirurgiegeräts mit Autostart-Funktion. Die Schaltung 1 verfügt über einen HF-Generator 65 zum Erzeugen des HF-Stroms. In der Schaltung 1 sind ein erster Komparator 44, ein zweiter Komparator 50, ein dritter Komparator 55 und ein vierter Komparator 60 vorgesehen. Die Schaltung 1 verfügt über vier Ausgänge 15, an denen jeweils eine monopolare 70 oder bipolare Elektrode 71 angeschlossen ist. Jedem Anschluss 15 ist eine erste Messeinrichtung 5 zugeordnet.

Zuerst wird die Autostart-Funktion für einen oder mehrere Ausgänge 15 aktiviert, indem die jeweiligen ersten Messeinrichtungen 5 eines Ausgangs 15 oder mehrerer Ausgänge 15 eingeschaltet werden. Die Autostart-Funktion kann für alle Ausgänge 15 oder nur für einen Teil der Ausgänge 15 des HF-Chirurgiegeräts aktiviert werden. Die jeweilige erste Messeinrichtung 5 misst nach dem Einschalten der ersten Messeinrichtungen 5 über Verbindungsleitungen 75 einen ersten Messwert der Impedanz der jeweiligen an dem Ausgang 15 angeschlossenen Elektrode 70, 71, d.h. die Impedanz zwischen den Polen des jeweiligen Ausgangs 15.

Die Impedanz der an dem Ausgang 15 angeschlossenen Elektrode 70, 71 ist im Allgemeinen komplexwertig; sie kann jedoch auch nur einen Realteil oder nur einen Imaginärteil haben bzw. nur der Realteil oder Imaginärteil wird gemessen. Verschiedene Methoden zur Messung einer Impedanz einer Elektrode 70,71 bzw. der Impedanz zwischen den beiden Polen einer Elektrode 70, 71 sind dem Fachmann bekannt. Anstelle der Impedanz können die ersten Messeinrichtungen auch alternativ eine andere physikalische Kenngröße der jeweiligen Elektrode 70, 71 messen.

Die Messung der ersten Messwerte kann in zueinander disjunkten Zeitintervallen stattfinden. Dadurch können Störungen der ersten Messeinrichtungen 5 untereinander durch Querströme weitgehend vermindert werden.

Der erste Komparator 44 ist über Verbindungsleitungen 46 mit den ersten Messeinrichtungen 5 verbunden und vergleicht den ersten Messwert mit einem ersten Sollwert. Ist der erste Messwert gleich dem oder größer als der erste Sollwert, wird davon ausgegangen, dass keine Elektrode 70, 71 Gewebe berührt hat und es wird erneut von der jeweiligen ersten Messeinrichtung 5 ein erster Messwert der Impedanz der am jeweiligen Ausgang 15 angeschlossenen Elektrode 70,71 gemessen.

Der erste Sollwert kann für einen Teil der Ausgänge 15 oder für jeden Ausgang 15 jeweils unterschiedlich sein. Jedem Ausgang 15 kann, beispielsweise abhängig von dem angeschlossenen bzw. anzuschließenden Instrument, ein unterschiedlicher erster Sollwert zugeordnet werden. Der erste Komparator 44 vergleicht in diesem Fall den ersten Messwert des jeweiligen Ausgangs 15 mit dem dem jeweiligen Ausgang 15 zugeordneten ersten Sollwert.

Ist der erste Messwert kleiner als der erste Sollwert, so schaltet der erste Komparator 44 die ersten Messeinrichtungen 5 der Ausgänge 15 aus und wählt den Ausgang 15 aus, bei dem der erste Messwert als zeitlich frühester der ersten Messwerte kleiner als der vorher bestimmte erste Sollwert ist. Der erste Komparator 44 schaltet über eine Verbindungsleitung 48 eine zweite Messeinrichtung 10 des HF-Chirurgiegeräts für den ausgewählten Ausgang 15 ein. Die zweite Messeinrichtung 10 ist mit jedem der Ausgänge 15 über eine Verbindungsleitung 76 verbunden. Die zweite Messeinrichtung 10 misst dann einen zweiten Messwert der Impedanz der an dem ausgewählten Ausgang 15 angeschlossenen Elektrode 70, 71.

Die zweite Messeinrichtung 10 kann eine qualitativ höherwertige Messeinrichtung als die ersten Messeinrichtungen 5 sein. Daher kann die zweite Messeinrichtung 10 einen genaueren Messwert der Impedanz der jeweiligen an dem ausgewählten Ausgang 15 angeschlossenen Elektrode 70,71 als die jeweilige erste Messeinrichtung 5 bestimmen. Zur genaueren Bestimmung der Impedanz der jeweiligen an dem ausgewählten Ausgang 15 angeschlossenen Elektrode 70, 71 verwendet die zweite Messeinrichtung 10 eine höhere Spannung als die ersten Messeinrichtungen 5. Durch die Verwendung einer niedrigeren Spannung zur Messung des ersten Messwerts durch die ersten Messeinrichtungen 5 werden die von dem HF-Chirurgiegerät ausgehenden Störungen anderer Geräte deutlich reduziert, da nur wenn der Gewebekontakt durch den ersten Komparator 50 festgestellt wurde, eine höhere Spannung wie z.B. von einigen Zehn Volt zur genaueren Messung der Impedanz der am ausgewählten Ausgang 15 angeschlossenen Elektrode 70, 71 verwendet wird.

Der zweite Komparator 50 ist über eine Verbindungsleitung 51 mit der zweiten Messeinrichtung 10 und über eine Verbindungsleitung 63 mit dem vierten Komparator 60 verbunden. Der zweite Komparator 50 vergleicht nun den zweiten Messwert mit einem zweiten Sollwert. Ist der zweite Messwert gleich dem oder größer als der zweite Sollwert, so vergleicht der vierte Komparator 60, der über Verbindungsleitungen 62 mit den ersten Messeinrichtungen 5 verbunden ist, den zweiten Messwert mit einem vierten Sollwert.

Ist der zweite Messwert kleiner als der vierte Sollwert, so wird der zweite Messwert der Impedanz der an dem ausgewählten Ausgang 15 angeschlossenen Elektrode 70, 71 mit der zweiten Messeinrichtung 10 erneut gemessen. Es wird in diesem Fall davon ausgegangen, dass das Gewebe nur kurz oder nur leicht berührt wurde.

Stellt der vierte Komparator 60 beim Vergleich fest, dass der zweite Messwert größer als der oder gleich dem vierten Sollwert ist, so schaltet der vierte Komparator 60 die zweite Messeinrichtung 10 über eine Verbindungsleitung 61 aus und die ersten Messeinrichtungen 5 ein. Die Schaltung 1 des HF-Chirurgiegeräts ist nun wieder in den Grundzustand zurückversetzt.

Stellt der zweite Komparator 50 beim Vergleich des zweiten Messwerts mit dem zweiten Sollwert fest, dass der zweite Messwert kleiner als der zweite Sollwert ist, so schaltet der zweite Komparator 50, der über eine Verbindungsleitung 66 mit dem HF-Generator 65 verbunden ist, den HF-Strom für den ausgewählten Ausgang 15 ein.

Die zweite Messeinrichtung 10 misst nach Einschalten des HF-Stroms erneut den zweiten Messwert der Impedanz der an dem ausgewählten Ausgang 15 angeschlossenen Elektrode 70, 71. Der dritte Komparator 55, der über eine Verbindungsleitung 56 mit der zweiten Messeinrichtung 10 und über Verbindungsleitungen 57 mit den Ausgängen 15 verbunden ist, vergleicht den zweiten Messwert mit einem dritten Sollwert. Ist der zweite Messwert nun kleiner dem dritten Sollwert so bleibt der HF-Strom eingeschaltet und es wird solange erneut der zweite Messwert der Impedanz der an dem ausgewählten Ausgang 15 angeschlossenen Elektrode 70, 71 mit der zweiten Messeinrichtung 10 gemessen, bis der zweite Messwert größer als oder gleich dem dritten Sollwert ist.

Stellt der dritte Komparator 55 fest, dass der zweite Messwert gleich dem oder größer als der dritte Sollwert ist, so schaltet der dritte Komparator 55 den HF-Strom über eine Verbindungsleitung 67 und die zweite Messeinrichtung 10 über eine Verbindungsleitung 56 aus und die ersten Messeinrichtungen 5 für die Ausgänge 15 werden eingeschaltet. Die Schaltung 1 des HF-Chirurgiegeräts ist nun wieder in den Grundzustand zurückversetzt.

Wenn die Elektrode 70, 71 biologisches Gewebe berührt, erniedrigt sich die Impedanz der Elektrode 70, 71 gegenüber der Impedanz bei Nicht-Kontakt zwischen Elektrode 70, 71 und biologischem Gewebe. Solange die Elektrode 70, 71 in Kontakt mit dem biologischen Gewebe steht, bleibt der HF-Strom daher eingeschaltet. Sobald kein Kontakt mehr zwischen Elektrode 70, 71 und biologischen Gewebe besteht, steigt die Impedanz an und liegt dann über dem dritten Sollwert, was zum Ausschalten des HF-Stroms und zum Zurückversetzten der Schaltung 1 des HF-Chirurgiegeräts in den Grundzustand führt.

Der dritte Sollwert und der vierte Sollwert können gleich groß sein. Dies führt dazu, dass die Schwelle, bei der die Schaltung 1 des HF-Chirurgiegeräts in den Grundzustand zurückversetzt wird, gleich groß ist, unabhängig davon, ob der zweite Messwert je kleiner als der zweite Sollwert war.

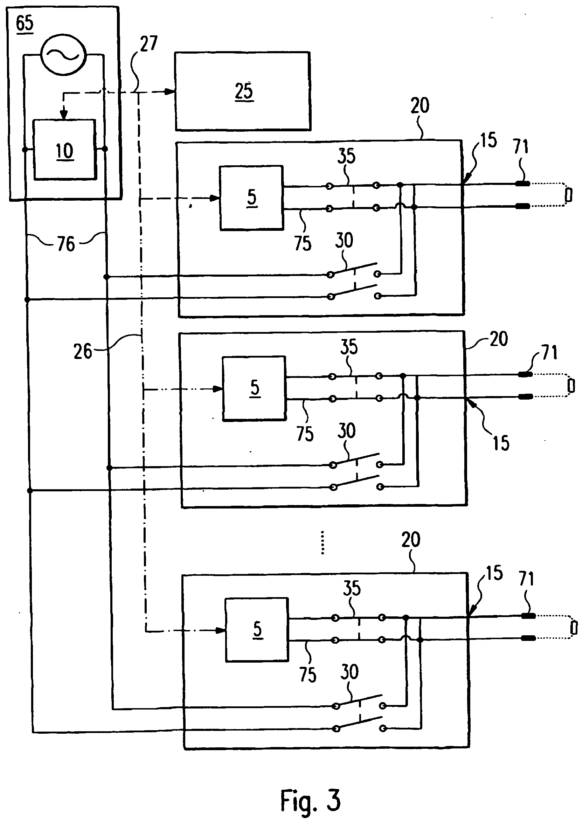

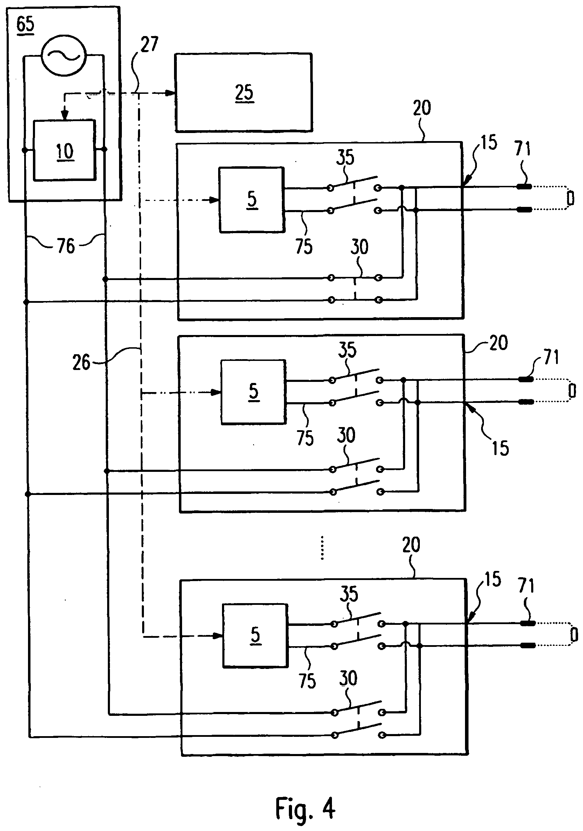

Fig. 3 und Fig. 4 zeigen eine schematische Ansicht einer Schaltung 1 eines HF-Chirurgiegeräts. Die Schaltung 1 umfasst einen HF-Generator 65 zum Erzeugen des HF-Stroms, ein Steuergerät 25, erste Messeinrichtungen 5, eine zweite Messeinrichtung 10 und mehrere Ausgänge 15 für jeweils eine bipolare Elektrode 71. Die zweite Messeinrichtung 10 ist im HP-Generator 65 angeordnet. Die zweite Messeinrichtung 10 überwacht zusätzlich die Betriebsdaten des HF-Generators 65. Jedem Ausgang 15 ist eine Ausgangseinheit 20 zugeordnet, die die jeweilige erste Messeinrichtung 5, ein jeweiliges HF-Relais 30 und ein jeweiliges Mess-Relais 35 umfasst. Die ersten Messeinrichtungen 5 sind jeweils über Verbindungsleitungen 75 über ein Messrelais 35 mit den Ausgängen 15 verbindbar. Der HF-Generator 65 bzw. die zweite Messeinrichtung 10 ist über Verbindungsleitungen 76 über ein HF-Relais 30 mit den Ausgängen 15 verbindbar.

In Fig. 3 sind die Mess-Relais 35 geschlossen, d.h. die ersten Messeinrichtungen 5 sind jeweils mit dem Ausgang 15 verbunden zur Messung der Impedanz der an dem jeweiligen Ausgang 15 angeschlossenen Elektrode 71, d.h. der Impedanz zwischen den Polen des jeweiligen Ausgangs 15. Liegt der von den ersten Messeinrichtungen 5 jeweils gemessene erste Messwert unterhalb eines ersten Sollwerts, wird der jeweilige Ausgang 15, bei dem der erste Messwert als zeitlich frühester der ersten Messwerte unter dem zweiten Sollwert liegt, vom Steuergerät 25 ausgewählt, das über eine Verbindungsleitung 26 mit den ersten Messeinrichtungen 5 verbunden ist.

Wie in Fig. 4 gezeigt werden die ersten Messeinrichtungen 5 durch das Steuergerät 25 ausgeschaltet, indem die Mess-Relais 35 geöffnet werden. Das HF-Relais 30 wird durch das Steuergerät 25, das über eine Verbindungsleitung 27 mit der zweiten Messeinrichtung 10 verbunden ist, für den ausgewählten Ausgang 15 geschlossen, so dass die zweite Messeinrichtung 10 und der HF-Generator 65 mit dem ausgewählten Ausgang 15 verbunden sind. Mit der zweiten Messeinrichtung 10 wird ein zweiter Messwert der Impedanz der an dem ausgewählten Ausgang 15 angeschlossenen Elektrode 71 bestimmt. Liegt der zweite Messwert unterhalb eines zweiten Sollwerts, so wird der HF-Strom für den ausgewählten Ausgang eingeschaltet. Während der HF-Strom eingeschaltet ist, wird immer wieder der zweite Messwert erneut gemessen und mit einem dritten Sollwert verglichen. Sobald der zweite Messwert oberhalb des dritten Sollwerts liegt, d.h. der Kontakt zwischen Elektrode 71 und Gewebe gelöst wurde oder das Gewebe durch fortschreitende Koagulation seine Impedanz erhöht hat, wird der HF-Strom ausgeschaltet, die zweite Messeinrichtung 10 ausgeschaltet, indem das HF-Relais 30 geöffnet wird, und die ersten Messeinrichtungen 5 erneut eingeschaltet, indem die Mess-Relais 35 geschlossen werden.

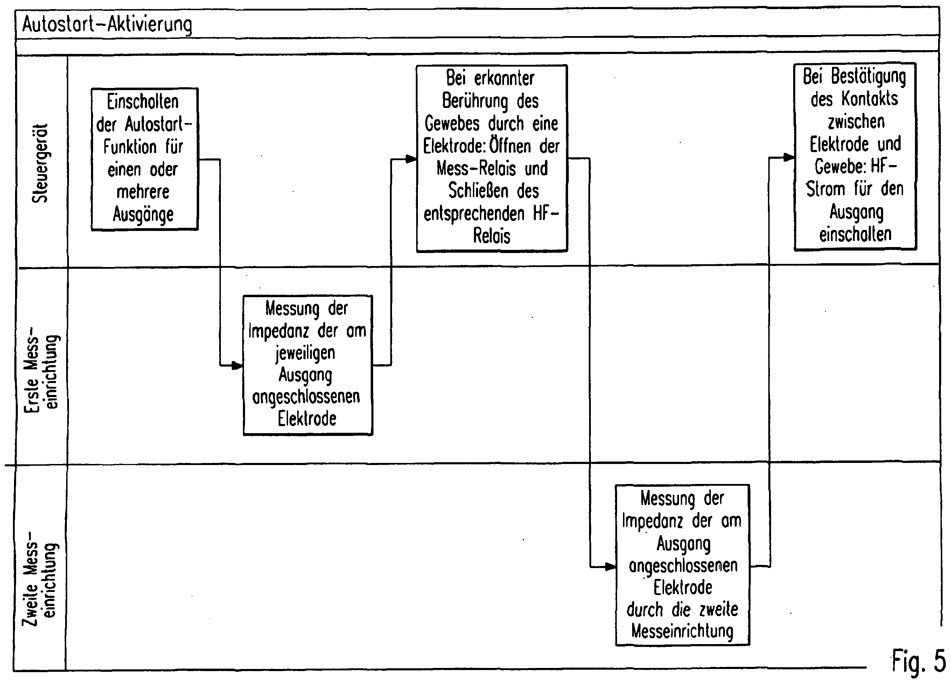

Fig. 5 zeigt ein Flussdiagramm für den Betrieb der Schaltung 1 nach Fig. 3 bzw. Fig. 4. Das Steuergerät 25 schaltet die Autostart-Funktion für einen oder mehrere Ausgänge 15 ein. Die jeweiligen ersten Messeinrichtungen 5 messen die Impedanz der am jeweiligen Ausgang 15 angeschlossenen Elektrode 70, 71. Wird eine Berührung einer Elektrode mit dem Gewebe festgestellt, so öffnet das Steuergerät 25 die Mess-Relais 35 und schließt das HF-Relais 30 für den Ausgang 15, bei dem als zeitlich ersten eine Berührung durch ein Unterschreiten eines Sollwerts der Impedanz festgestellt wurde. Die zweite Messeinrichtung 10 misst nun erneut die Impedanz der am durch das HF-Relais 35 verbundenen Ausgang 15 angeschlossenen Elektrode 70, 71. Bestätigt nun das Steuergerät 25 durch ein Vergleich des zweiten Messwerts mit einem zweiten Sollwert, dass die Elektrode 70, 71 Gewebe berührt, so wird der HF-Strom für die Elektrode 70, 71 eingeschaltet.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste:

- 1: Schaltung
- 5: erste Messeinrichtungen
- 10: zweite Messeinrichtung
- 15: Ausgang
- 20: Ausgangseinheit
- 25: Steuergerät
- 30: HF-Relais
- 35: Mess-Relais
- 44: erster Komparator
- 46: Verbindungsleitungen erster Komparator-erste Messeinrichtungen
- 47: Verbindungsleitung erster Komparator-zweiter Komparator
- 48: Verbindungsleitung erster Komparator-zweite Messeinrichtung
- 50: zweiter Komparator
- 51: Verbindungsleitung zweiter Komparator-zweite Messeinrichtung
- 55: dritter Komparator
- 56: Verbindungsleitung dritter Komparator-zweite Messeinrichtung
- 57: Verbindungsleitungen dritter Komparator-erste Messeinrichtungen
- 58: Verbindungsleitung dritter Komparator-zweiter Komparator
- 60: vierter Komparator
- 61: Verbindungsleitung vierter Komparator-zweite Messeinrichtung
- 62: Verbindungsleitungen vierter Komparator-erste Messeinrichtungen
- 63: Verbindungsleitung vierter Komparator-zweiter Komparator
- 65: HF-Generator
- 66: Verbindungsleitung HF-Generator-zweiter Komparator
- 67: Verbindungsleitung HF-Generator-dritter Komparator
- 68: Verbindungsleitung HF-Generator-Anschlüsse
- 70: monopolare Elektrode
- 71: bipolare Elektrode
- 75: Verbindungsleitungen erste Messeinrichtungen-Anschlüsse
- 76: Verbindungsleitung zweite Messeinrichtung-Anschlüsse

## Patentansprüche

1. HF-Chirurgiegerät mit einer Autostart-Funktion zum Schneiden und/oder Koagulieren von biologischem Gewebe mit HF-Strom, umfassend:
- einen HF-Generator (65) zum Erzeugen des HF-Stroms,
- einen ausgewählten Ausgang (15) zum Anschließen einer monopolaren oder bipolaren Elektrode (70, 71) an das HF-Chirurgiegerät,
- eine dem Ausgang (15) zugeordnete erste Messeinrichtung (5) zum Messen eines ersten Messwerts einer Impedanz der an dem Ausgang (15) angeschlossenen Elektrode (70, 71),
- einen ersten Komparator (44) zum Vergleichen des ersten Messwerts mit einem ersten Sollwert,
**dadurch gekennzeichnet, dass**
der erste Komparator (44) ferner zum Ausschalten der ersten Messeinrichtungen (5) und zum Einschalten einer zweiten Messeinrichtung (10), wenn der erste Messwert kleiner als der erste Sollwert ist, zum Messen eines zweiten Messwerts der Impedanz für die angeschlossene Elektrode (70, 71) eingerichtet ist und
- dass das HF-Chirurgiegerät einen zweiten Komparator (50) zum Vergleichen des zweiten Messwerts mit einem zweiten Sollwert und zum Einschalten des HF-Stroms für den Ausgang (15), wenn der zweite Messwert kleiner als der zweite Sollwert ist, umfasst,
wobei die erste Messeinrichtung (5) im Vergleich zur zweiten Messeinrichtung (10) eine geringere Spannung zur Messung der Impedanz verwendet,
wobei die zweite Messeinrichtung (10) einen genaueren Messwert der Impedanz der jeweiligen an,dem ausgewählten Ausgang (15) angeschlossenen Elektrode (70, 71) bestimmt als die jeweilige erste Messeinrichtung (5),
wobei die zweite Messeinrichtung (10) in dem HF-Generator (65) zum Erzeugen des HF- Stroms angeordnet ist.

2. HF-Chirurgiegerät nach Anspruch 1 mit
- mindestens zwei Ausgängen (15) zum Anschließen von monopolaren und/oder bipolaren Elektroden (70, 71) an das HF-Chirurgiegerät,
- jeweils eine dem jeweiligen Ausgang (15) zugeordnete erste Messeinrichtung (5) zum Messen eines ersten Messwerts einer Impedanz der jeweiligen an dem Ausgang (15) angeschlossenen Elektrode (70, 71),
- wobei der erste Komparator (44) zum Auswählen eines Ausganges (15) eingerichtet ist, bei dem der erste Messwert als zeitlich frühester der ersten Messwerte kleiner als der vorher bestimmte erste Sollwert ist.

3. HF-Chirurgiegerät nach einem der vorhergehenden Ansprüche **gekennzeichnet durch** einen dritten Komparator (55) zum Vergleichen des zweiten Messwerts mit einem dritten Sollwert und zum Ausschalten des HF-Stroms und der zweiten Messeinrichtung (10) und zum Einschalten der ersten Messeinrichtungen (5), wenn der zweite Messwert größer als der oder gleich dem dritten Sollwert ist.

4. HF-Chirurgiegerät nach einem der vorhergehenden Ansprüche **gekennzeichnet durch** einen vierten Komparator (60) zum Vergleichen des zweiten Messwerts mit einem vierten Sollwert und zum Ausschalten der zweiten Messeinrichtung (10) und zum Einschalten der ersten Messeinrichtungen (5), wenn der zweite Messwert größer als der oder gleich dem vierten Sollwert ist.

5. HF-Chirurgiegerät nach einem der vorhergehenden Ansprüche, insbesondere nach Anspruch 4, wobei der dritte und vierte Sollwert gleich groß sind.

6. HF-Chirurgiegerät nach einem der vorhergehenden Ansprüche, wobei die ersten Messeinrichtungen (5) derart ausgebildet sind, dass sie die ersten Messwerte in zueinander disjunkten Zeitintervallen messen.

## Claims

1. HF surgical device having an autostart function for cutting and/or coagulating biological tissue by means of HF current, comprising
- an HF generator (65) for producing the HF current,
- a selected output (15) for connecting a monopolar or bipolar electrode (70, 71) to the HF surgical device,
- a first measuring device (5) associated with the output (15) for measuring a first measured value of an impedance of the electrode (70, 71) connected to the output (15),
- a first comparator (44) for comparing the first measured value to a first nominal value,
**characterised in that**
the first comparator (44) is furthermore configured to switch off the first measuring devices (5) and switch on a second measuring device (10) if the first measured value is less than the first nominal value, for measuring a second measured value of the impedance for the connected electrode (70, 71), and
- that the HF surgical device has a second comparator (50) for comparing the second measured value with a second nominal value and for switching on the HF current for the output (15) if the second measured value is less than the second nominal value, wherein the first measurement device (5) uses a lower voltage for measuring the impedance than the second measurement device (10),
wherein the second measurement device (10) determines a more precise measured value of the impedance of the respective electrode (70, 71) connected to the selected output (15) than the respective first measurement device (5),
wherein the second measurement device (10) is arranged in the HF generator (65) for generating HF current.

2. HF surgical device according to claim 1, with
- at least two outputs (15) for connecting monopolar and/or bipolar electrodes (70, 71) to the HF surgical device,
- a respective first measuring device (5) associated with the respective output (15) for measuring a first measured value of an impedance of the respective electrode (70, 71) connected to the output (15),
- wherein the first comparator (44) is configured to select an output (15) at which the first measurement value, as the temporally earliest of the first measurement values, is lower than the first nominal value previously determined.

3. HF surgical device according to any of the preceding claims, **characterised by** a third comparator (55) for comparing the second measured value with a third nominal value, and for switching off the HF current and the second measurement device (10) and for switching on the first measurement devices (5) if the second measured value is greater than or equal to the third nominal value.

4. HF surgical device according to any of the preceding claims, **characterised by** fourth comparator (60) for comparing the second measured value with a fourth nominal value and for switching off the second measurement device (10) and switching on the first measurement devices (5) if the second measured value is greater than or equal to the fourth nominal value.

5. HF surgical device according to any of the preceding claims, in particular claim 4, wherein the third and fourth nominal values are equally great.

6. HF surgical device according to any of the preceding claims, wherein the first measurement devices (5) are configured such that they measure the first measurement values at mutually separated time intervals.

## Revendications

1. Appareil chirurgical HF à fonction d'activation automatique (autostart) pour la coupe et/ou la coagulation de tissus biologiques par courant HF, comprenant :
- un générateur HF (65) pour la production du courant HF,
- une sortie (15) sélectionnée pour la connexion d'une électrode (70, 71) monopolaire ou bipolaire à l'appareil chirurgical HF,
- un premier dispositif de mesure (5) associé à la sortie (15) pour la mesure d'une première valeur mesurée d'une impédance de l'électrode (70, 71) connectée à la sortie (15),
- un premier comparateur (44) pour la comparaison de la première valeur mesurée avec une première valeur de consigne,
**caractérisé en ce que**
le premier comparateur (44) est en outre conçu pour la mise hors circuit des premiers dispositifs de mesure (5) et pour la mise en circuit d'un deuxième dispositif de mesure (10), lorsque la première valeur mesurée est plus petite que la première valeur de consigne, pour la mesure d'une deuxième valeur mesurée pour l'électrode (70, 71) connectée, et
- **en ce que** l'appareil chirurgical HF comprend un deuxième comparateur (50) pour la comparaison de la deuxième valeur mesurée avec une deuxième valeur de consigne et pour la mise en circuit du courant HF pour la sortie (15), lorsque la deuxième valeur mesurée est plus petite que la deuxième valeur de consigne,
sachant que le premier dispositif de mesure (5), comparé au deuxième dispositif de mesure (10), utilise une tension plus faible pour la mesure de l'impédance,
sachant que le deuxième dispositif de mesure (10) détermine une valeur mesurée de l'impédance de l'électrode (70, 71) respective connectée à la sortie (15) sélectionnée, qui est plus précise que celle du premier dispositif de mesure (5) respectif,
sachant que le deuxième dispositif de mesure (10) est installé dans le générateur HF (65) pour la production du courant HF.

2. Appareil chirurgical HF selon la revendication 1, comprenant
- au moins deux sorties (15) pour la connexion d'électrodes (70, 71) monopolaires et/ou bipolaires à l'appareil chirurgical HF,
- respectivement un premier dispositif de mesure (5) associé à la sortie (15) respective pour la mesure d'une première valeur mesurée d'une impédance de l'électrode (70, 71) respective connectée à la sortie (15),
- sachant que le premier comparateur (44) est conçu pour la sélection d'une sortie (15) où la première valeur mesurée, en tant que valeur mesurée obtenue le plus tôt dans le temps, est plus petite que la première valeur de consigne définie précédemment.

3. Appareil chirurgical HF selon l'une des revendications précédentes, **caractérisé par** un troisième comparateur (55) pour la comparaison de la deuxième valeur mesurée avec une troisième valeur de consigne et pour la mise hors circuit du courant HF et du deuxième dispositif de mesure (10) et pour la mise en circuit des premiers dispositifs de mesure (5), lorsque la deuxième valeur mesurée est plus grande ou égale à la troisième valeur de consigne.

4. Appareil chirurgical HF selon l'une des revendications précédentes, **caractérisé par** un quatrième comparateur (60) pour la comparaison de la deuxième valeur mesurée avec une quatrième valeur de consigne et pour la mise hors circuit du deuxième dispositif de mesure (10) et pour la mise en circuit des premiers dispositifs de mesure (5), lorsque la deuxième valeur mesurée est plus grande ou égale à la quatrième valeur de consigne.

5. Appareil chirurgical HF selon l'une des revendications précédentes, en particulier selon la revendication 4, où la troisième et la quatrième valeur de consigne sont égales.

6. Appareil chirurgical HF selon l'une des revendications précédentes, où les premiers dispositifs de mesure (5) sont agencés de manière telle qu'ils mesurent les premières valeurs mesurées à des intervalles de temps qui sont disjonctifs les uns par rapport aux autres.
